# EUROPÄISCHE PATENTSCHRIFT

(11) **EP 1 086 092 B1**
(45) Veröffentlichungstag und Bekanntmachung des Hinweises auf die Patenterteilung: **13.11.2002**
(21) Anmeldenummer: 99927784.1
(22) Anmeldetag: 28.05.1999
(51) Int. Cl.: C07D 337/08, C07K 5/068, A61K 31/38

(54) **BENZO(b)THIEPIN-1,1-DIOXIDDERIVATE, VERFAHREN ZU DEREN HERSTELLUNG, DIESE VERBINDUNGEN ENTHALTENDE ARZNEIMITTEL UND DEREN VERWENDUNG**
BENZO(b)THIEPINE-1,1-DIOXIDE DERIVATIVES, A METHOD FOR THE PRODUCTION THEREOF, MEDICAMENTS CONTAINING THESE COMPOUNDS, AND THEIR USE
DERIVES DE BENZO(b)THIEPINE-1,1-DIOXYDE, LEUR PROCEDE DE PREPARATION, MEDICAMENTS CONTENANT CES COMPOSES, ET LEUR UTILISATION

(30) Priorität: 10.06.1998 DE 19825804
(43) Veröffentlichungstag der Anmeldung: 28.03.2001
(73) Patentinhaber: Aventis Pharma Deutschland GmbH, 65929 Frankfurt am Main (DE)
(72) Erfinder: FRICK, Wendelin, D-65510 Hünstetten-Beuerbach (DE); ENHSEN, Alfons, D-64572 Büttelborn (DE); GLOMBIK, Heiner, D-65719 Hofheim (DE); HEUER, Hubert, D-55270 Schwabenheim (DE)
(86) Internationale Anmeldenummer: EP9903701
(87) Internationale Veröffentlichungsnummer: WO99064410

(56) Entgegenhaltungen:
- WO-A-97/33882

## Beschreibung

Die Erfindung betrifft substituierte Benzo(b)thiepin-1,1-dioxidderivate, deren physiologisch verträgliche Salze und physiologisch funktionelle Derivate.

Es sind bereits Benzo(b)thiepin-1,1-dioxidderivate sowie deren Verwendung zur Behandlung von Hyperlipidämie sowie Arteriosklerose und Hypercholesterinämie beschrieben worden [vgl. PCT Anmeldung Nr. PCT/US97/04076, Veröffentlichungs-Nr. WO 97/33882].

Der Erfindung lag die Aufgabe zugrunde, weitere Verbindungen zur Verfügung zu stellen, die eine therapeutisch verwertbare hypolipidämische Wirkung entfalten. Insbesondere bestand die Aufgabe darin, neue Verbindungen zu finden, die gegenüber den im Stand der Technik beschriebenen Verbindungen, bereits bei einer niedrigeren Dosierung eine höhere fäkale Gallensäureauscheidung bewirken. Eine Dosierungsreduzierung des ED₂₀₀ Wertes um mindestens den Faktor 5 gegenüber den im Stand der Technik beschriebenen Verbindungen war besonders wünschenswert.

Die Erfindung betrifft daher Verbindungen der Formel I, 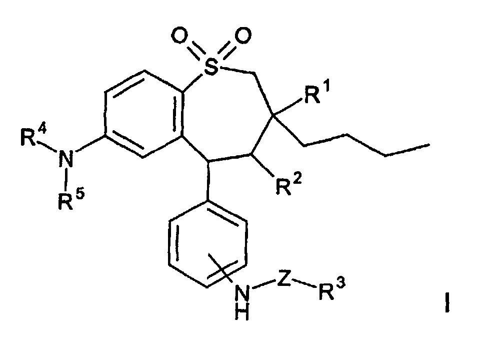 worin bedeuten
- R¹: Ethyl, Butyl;
- R²: OH;
- R³: Diaminosäurerest, wobei der Diaminosäurerest gegebenenfalls ein oder mehrfach substituiert ist durch eine Aminosäure-Schutzgruppe;
- R⁴: Methyl;
- R⁵: Methyl;
- Z: -(C=O)-C₀-C₄-Alkyl, eine kovalente Bindung;
sowie deren pharmazeutisch verträgliche Salze.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natriumund Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Benzo(b)thiepin-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säureund magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Gegenstand der Erfindung sind weiterhin sowohl Isomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

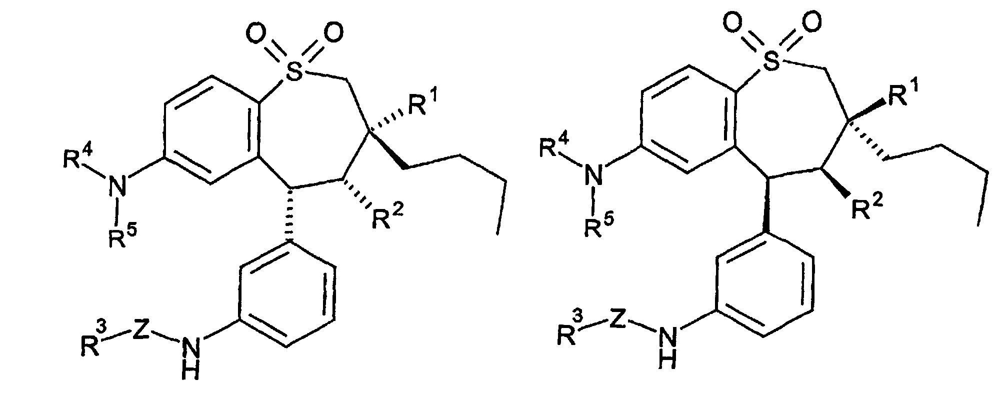

Mit dem Begriff Aminosäuren bzw. Aminosäurereste sind z.B. die stereoisomeren Formen, d.h. D- oder L-Formen, folgender Verbindungen gemeint:

| | | |
|---|---|---|
| Alanin | Glycin | Prolin |
| Cystein | Histidin | Glutamin |
| Asparaginsäure | Isoleucin | Arginin |
| Glutaminsäure | Lysin | Serin |
| Phenylalanin | Leucin | Threonin |
| Tryptophan | Methionin | Valin |
| Tyrosin | Asparagin | |

| | |
|---|---|
| 2-Aminoadipinsäure | 2-Aminoisobuttersäure |
| 3-Aminoadipinsäure | 3-Aminoisobuttersäure |
| beta-Alanin | 2-Aminopimelinsäure |
| 2-Aminobuttersäure | 2,4-Diaminobuttersäure |
| 4-Aminobuttersäure | Desmosin |
| Piperidinsäure | 2,2-Diaminopimelinsäure |
| 6-Aminocapronsäure | 2,3-Diaminopropionsäure |
| 2-Aminoheptansäure | N-Ethylglycin |
| 2-(2-Thienyl)-glycin | 3-(2-Thienyl)-alanin |
| Penicillamin | Sarkosin |
| N-Ethylasparagin | N-Methylisoleucin |
| Hydroxylysin | 6-N-Methyllysin |
| allo-Hydroxylysin | N-Methylvalin |
| 3-Hydroxyprolin | Norvalin |
| 4-Hydroxyprolin | Norleucin |
| Isodesmosin | Omithin |
| allo-lsoleucin | |
| N-Methylglycin | |

Die Kurzschreibweise der Aminosäuren erfolgte nach der allgemein üblichen Schreibweise (vgl. Schröder, Lübke, The Peptides, Band I, New York 1965, Seiten XXII-XXIII; Houben-Weyl, Methoden der Organischen Chemie, Band XV/1 und 2, Stuttgart 1974). Die Aminosäure pGlu steht für Pyroglutamyl, Nal für 3-(2-Naphthyl)-alanin, Azagly-NH₂ für eine Verbindung der Formel NH₂-NH-CONH₂ und D-Asp für die D-Form von Asparaginsäure. Peptide sind ihrer chemischen Natur nach Säureamide und zerfallen bei der Hydrolyse in Aminosäuren.

Unter Diaminosäurerest versteht man Peptide, die aus 2 der oben genannten Aminosäuren aufgebaut sind.

Geeignete Schutzgruppen (siehe z.B. T.W. Greene, "Protective Groups in Organic Synthesis") für Aminosäuren sind in erster Linie:
Arg(Tos), Arg(Mts), Arg(Mtr), Arg(PMV), Asp(OBzl), Asp(OBut), Cys(4-MeBzl), Cys(Acm), Cys(SBut), Glu(Obzl), Glu(Obut), His(Tos), His(Fmoc), His(Dnp), His(Trt), Lys(CI-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl- (Trt)-Rest und der durch sekundäre Amine abspaltbare 9-Fluorenylmethyloxycarbonyl- (Fmoc)-Rest herangezogen.

Die Erfindung betrifft weiterhin Verfahren zur Herstellung von Benzo(b)thiepin-1,1-dioxidderivate der Formel I:

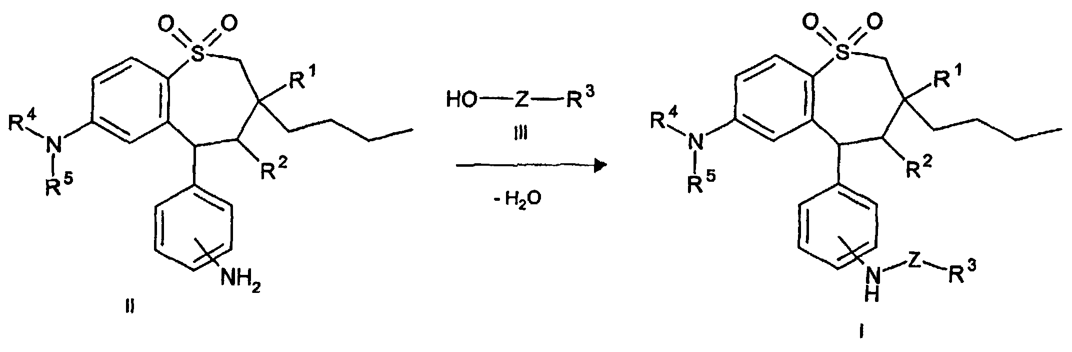

Verfahren zur Herstellung der Verbindungen der Formel I, dadurch gekennzeichnet, daß man ein Amin der Formel II, in der R¹, R², R⁴ und R⁵ die zu Formel I angegebenen Bedeutungen haben, mit einer Verbindung der Formel III, in der R³ und Z die zu Formel I angegebenen Bedeutungen haben, unter Wasserabspaltung zu einer Verbindung der Formel I umsetzt und die erhaltene Verbindung der Formel I gegebenenfalls in ein physiologisch verträgliches Salz überführt. Wenn es sich bei dem Rest R³ um eine Monoaminosäure handelt, kann dieser Rest gegebenenfalls auch noch nach der Bindung an das Amin der Formel II stufenweise zum Diaminosäurerest, Triaminosäurerest oder Tetraaminosäurerest verlängert werden.

Die Verbindungen der Formel I und deren pharmazeutisch verträgliche Salze stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen. Die Verbindungen können gegebenenfalls auch in Kombination mit Statinen, wie z.B. Simvastatatin, Fluvastatin, Pravastatin, Cerivastatin, Lovastatin oder Atorvastin verabreicht werden. Folgende Befunde belegen die pharmakologische Wirksamkeit der erfindungsgemäßen Verbindungen.

Die biologische Prüfung der erfindungsgemäßen Verbindungen erfolgte durch Ermittlung der ED₂₀₀ Ausscheidung. Diese Prüfung untersucht die Wirkung der erfindungsgemäßen Verbindungen auf den Gallensäurentransport im Ileum und die fäkale Ausscheidung von Gallensäuren bei der Ratte nach oraler Verabreichung zweimal täglich. Es wurden die Diastereomerengemische der Verbindungen geprüft.

### Der Test wurde wie folgt durchgeführt:

### 1) Zubereitung der Test- und Referenzsubstanzen

Zur Formulierung einer wässerigen Lösung diente folgende Rezeptur: Die Substanzen wurden in adäquatem Volumen einer Solutol (=Polyethylenglykol 600 Hydroxystearat; BASF, Ludwigshafen,Deutschland; Chargennr. 1763) enthaltenden wässrigen Lösung gelöst, so daß eine Endkonzentration von 5% Solutol in der wässerigen Lösung vorliegt. Die Lösungen/Suspensionen wurden in einer Dosierung von 5ml/kg per os verabreicht.

### 2) Versuchsbedingungen

Männliche Wistar Ratten (Kastengrund, Hoechst AG, Gewichtsbereich 250-350g) wurden in Gruppen zu jeweils 6 Tieren und ab 10 Tagen vor Behandlungsbeginn (Tag 1) bei einem umgekehrten Tag/Nacht Rhythmus (4^{oo}-16^{oo} dunkel, 16^{oo} -4^{oo} hell) gehalten und erhielten eine Standard Futtermischung (Altromin, Lage, Deutschland). Drei Tage vor Versuchsbeginn (Tag 0) wurden die Tiere in Gruppen mit jeweils 4 Tieren eingeteilt.

### Einteilung der Tiere in Behandlungsgruppen:

| Nummer der Gruppe | Tiernr. / Analysennr. | Testsubstanz ¹ | Dosis (mg/kg/d) |
|---|---|---|---|
| | | | |
| 1 | 1-4 | negative Kontrolle | Trägersubstanz |
| 2 | 5-8 | Testsubstanz Dosis 1 | 2 x 0,008 |
| 3 | 9-12 | Testsubstanz Dosis 2 | 2 x 0,02 |
| 4 | 13-16 | Testsubstanz Dosis 3 | 2 x× 0,1 |
| 5 | 17-20 | Testsubstanz Dosis 4 | 2 x 0,5 |

| | | | |
|---|---|---|---|
| ¹ gelöst/suspendiert in 5% Solutol HS 15/0,4% Stärkeschleim | | | |

### 3) Versuchsablauf

Nach intravenöser oder subkutaner Verabreichung von 5 µCi ¹⁴C-Taurocholat pro Ratte (Tag 0) wurden die Träger- oder Testsubstanzen um 7^{oo} -8^{oo} und um 15^{oo}-16^{oo} des folgenden Tages (Tag 1) gegeben (Behandlung für einen Tag). Kotproben für die Analyse von ¹⁴C-Taurocholat wurden alle 24 Stunden direkt nach der Verabreichung der morgendlichen Dosis genommen. Die Fäzes wurden gewogen, bei -18°C gelagert und später in 100 ml Aqua demineralisata suspendiert und homogenisiert (Ultra Turrax, Janke & Kunkel, IKA-Werk). Aliquote Teile (0,5 g) wurden gewogen und auf Verbrennungshütchen (Combusto Cones, Canberra Packard) in einer Verbrennungsapparatur (Tri Carb® 307 combuster Canberra Packard GmbH, Frankfurt am Main, Deutschland) verbrannt. Das entstandene ¹⁴CO₂ wurde mit Carbo-Sorb® (Canberra Packard) absorbiert. Die folgenden ¹⁴C Radioaktivitätsmessungen wurden nach Zugabe des Szintillators (Perma-Fluor complete scintillation cocktail Nr. 6013187, Packard) zu den Proben mit Hilfe der Flüssigszintillationszählung (LSC) bestimmt. Die fäkale Ausscheidung von ¹⁴C-Taurocholsäure wurde als kumulative und/oder prozentuale Restradioaktivität berechnet (siehe unten).

### 4) Beobachtungen und Messungen

Die fäkale Ausscheidung von ¹⁴C-TCA wurde in verbrannten aliquoten Teilen der in 24 Stunden-Intervallen genommenen Kotproben bestimmt, als "kumulativer Prozentsatz" der verabreichten Aktivität berechnet und als % der Restaktivität (=verbleibende Aktivität, d.h. verabreichte Aktivität abzüglich der bereits ausgeschiedenen Aktivität) ausgedrückt. Für die Berechnung der Dosis-Wirkungs-Kurven wurde die Ausscheidung von ¹⁴C Taurocholsäure als Prozentanteil der entsprechenden Werte der Kontrollgruppe (behandelt mit Trägersubstanz) ausgedrückt. Die ED₂₀₀, d.h. die Dosis, die die fäkale Ausscheidung von ¹⁴C Taurocholsäure auf 200% der Kontrollgruppe steigert, wird durch Interpolation aus einer sigmoiden oder linearen Dosis-Wirkungs-Kurve berechnet. Die kalkulierte ED₂₀₀ entspricht einer Dosis, die die fäkale Ausscheidung von Gallensäuren verdoppelt.

### 5) Ergebnisse

Tabelle 1 zeigt Meßwerte der ED₂₀₀ Ausscheidung.

**Tabelle 1:**

| Verbindungen aus Beispiel | ED₂₀₀ Ausscheidung (mg/kg/d) p.o. |
|---|---|
| 4 | 0,04 |
| | |
| Vergleichsbeispiele | |
| 1 | 0,8 |
| 2 | 1,0 |
| 3 | 0,9 |

### 6) Diskussion

Aus den Meßdaten ist abzulesen, daß die erfindungsgemäßen Verbindungen der Formel I gegenüber den im Stand der Technik beschriebenen Verbindungen eine um den Faktor 20 bessere Wirkung aufweisen.

Die nachfolgenden Beispiele dienen zur näheren Erläuterung der Erfindung, ohne dieselbe auf in den Beispielen beschriebene Produkte und Ausführungsformen einzuschränken.

### Beispiel 4

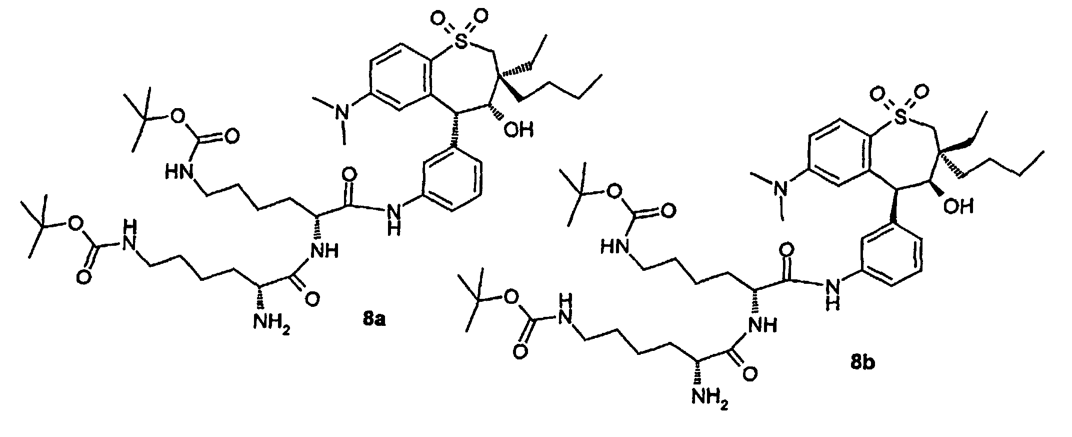

C₄₆H₇₄N₆O₉S (887.20). MS (M + H)⁺ = 887.5

Vergleichsbeispiele aus PCT/US97/04076:

### Vergleichsbeispiele 1

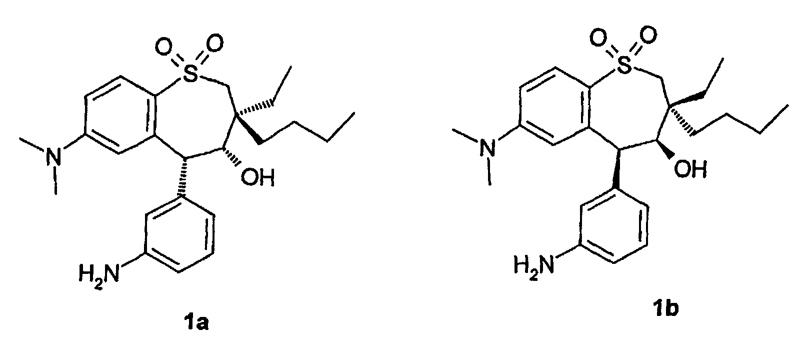

### Vergleichsbeispiele 2

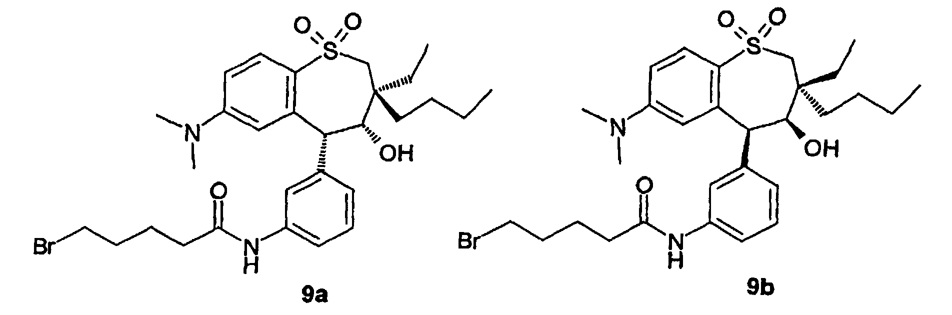

### Vergleichsbeispiele 3

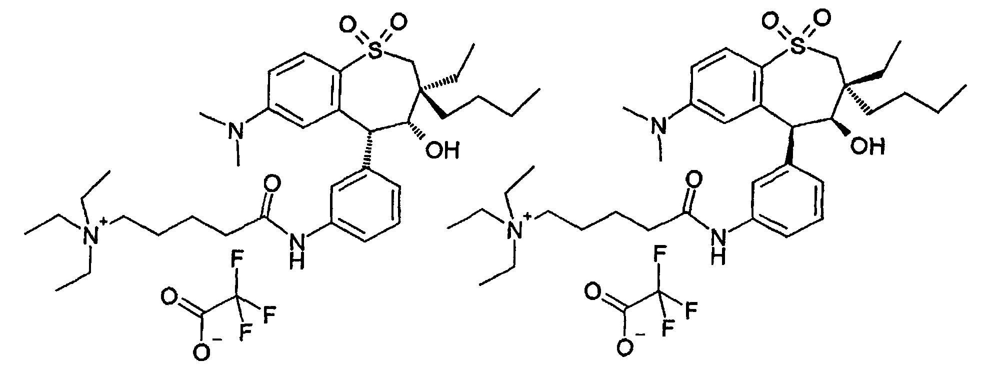

Die Beispiele bzw. Vergleichsbeispiele wurden wie folgt hergestellt (bei den Darstellungen wird nur die Synthese der a-Diastereomeren gezeigt):

### Formelschema 1

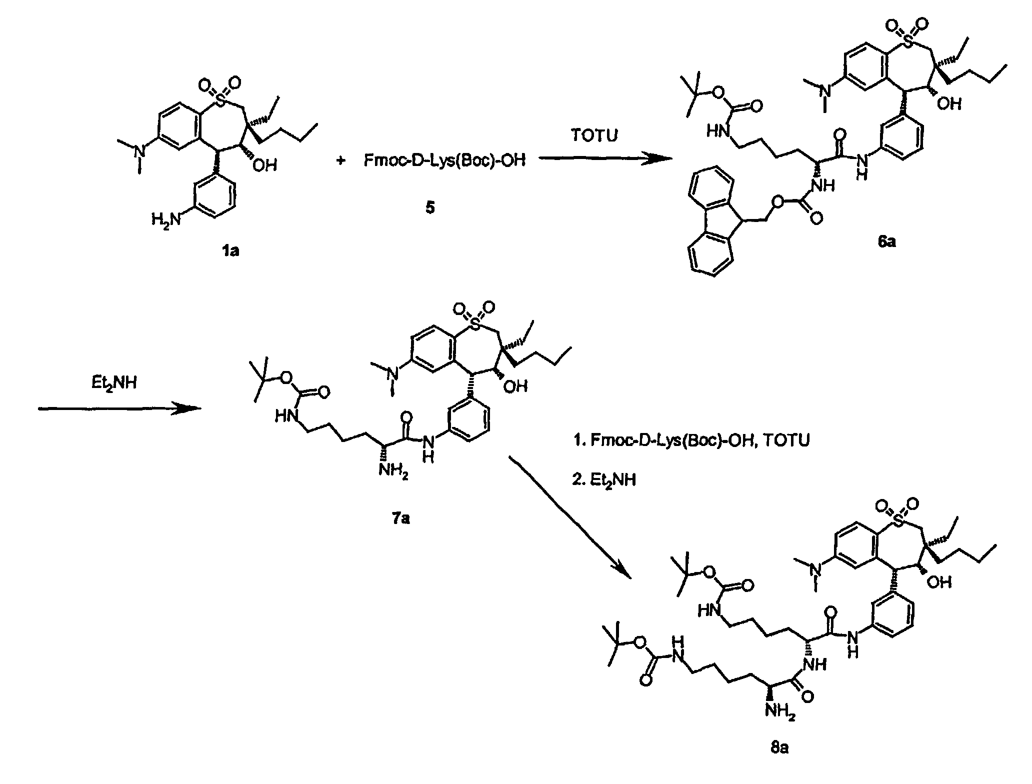

### Synthese von Verbindung 6 als Diastereomerengemisch:

150 mg (0.35 mmol) **1a/b** und 245 mg (0.52 mmol) Fmoc-D-Lys(Boc)-OH **5** (Fluka) in 6 ml DMF werden mit 169 mg TOTU, 74 mg Oxim und 0.5 ml NEM analog der Synthese von Verbindung **3** umgesetzt. Ausbeute 290 mg (94%) **6a/b** als amorpher Feststoff. DC (Ethylacetat/ n-Heptan 2:1). R_{f} = 0.6. C₅₀H₆₄N₄O₈S (881.15), MS (M + H)⁺ = 881.5.

### Synthese von Verbindung 7 als Diastereomerengemisch:

285 mg (0.32 mmol) **6a/b** werden in 5 ml DMF gelöst. Nach Zugabe von 0.6 ml Diethylamin läßt man 30 Minuten stehen. Die Aufarbeitung erfolgt analog der Synthese von Verbindung **3**. Ausbeute 173 mg (81 %) **7a/b** als amorpher Feststoff. DC ( Methylenchlorid/ Methanol 15:1). R_{f} = 0.2, Edukt **6a/b** R_{f}= 0.4. C₃₅H₅₄N₄O₆S (658.91). MS (M + H)⁺ = 659.4.

### Synthese von Verbindung 8 als Diastereomerengemisch:

168 mg (0.25 mmol) **7a/b** werden analog der Synthese von Verbindung **6** und **7** umgesetzt und man erhält 169 mg (75% über zwei Stufen) **8a/b** als amorpher Feststoff. DC (Methylenchlorid/ Methanol 9:1). R_{f} = 0.3. C₄₆H₇₄N₆O₉S (887.20). MS (M + H)⁺ = 887.5

## Patentansprüche

1. Verbindungen der Formel I, 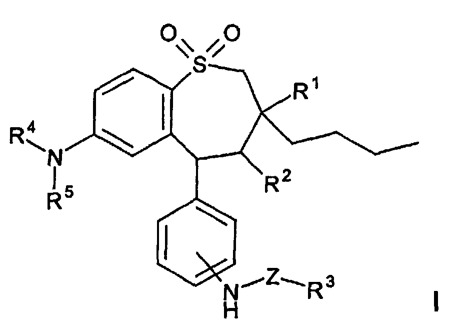 worin bedeuten
R¹ Ethyl, Butyl;
R² OH;
R³ Diaminosäurerest, wobei der Diaminosäurerest gegebenenfalls ein oder mehrfach substituiert ist durch eine Aminosäure-Schutzgruppe;
R⁴ Methyl;
R⁵ Methyl;
Z -(C=O)-C₀-C₄-Alkyl, eine kovalente Bindung;
sowie deren pharmazeutisch verträgliche Salze.

2. Verfahren zur Herstellung von Verbindungen der Formel I, gemäß Anspruch 1, **dadurch gekennzeichnet, daß** man nach folgendem Formelschema 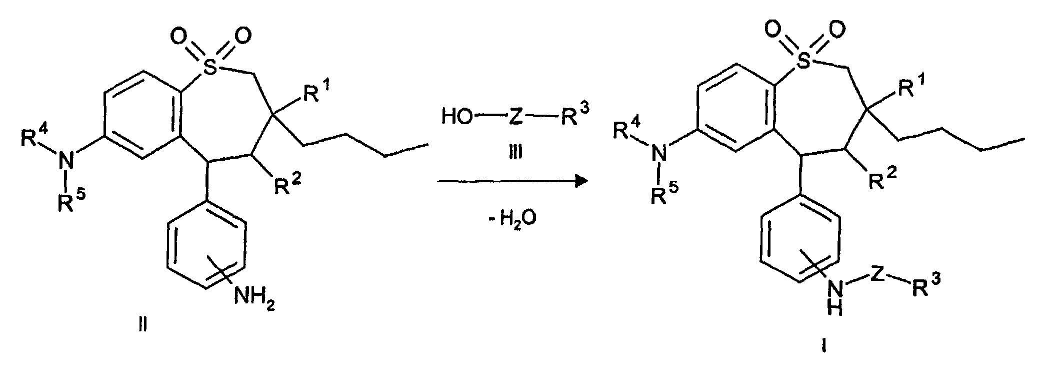 ein Amin der Formel II, in der R¹, R², R⁴ und R⁵ die zu Formel I angegebenen Bedeutungen haben, mit einer Verbindung der Formel III, in der R³ und Z die zu Formel I angegebenen Bedeutungen haben, unter Wasserabspaltung zu einer Verbindung der Formel I umsetzt und die erhaltene Verbindung der Formel I gegebenenfalls in ein physiologisch verträgliches Salz überführt.

3. Arzneimittel enthaltend eine oder mehrere der Verbindungen gemäß Anspruch 1.

4. Arzneimittel enthaltend eine oder mehrere der Verbindungen gemäß Anspruch 1 und ein oder mehrere Statine.

5. Verbindungen gemäß Anspruch 1 zur Herstellung eines Medikaments zur Behandlung von Lipidstoffwechselstörungen.

6. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

7. Verwendung der Verbindungen gemäß Anspruch 1 zur Herstellung eines Medikaments zur Beeinflussung des Serumcholesterinspiegels.

8. Verwendung der Verbindungen gemäß Anspruch 1 zur Herstellung eines Medikaments zur Prävention arteriosklerotischer Erscheinungen.

## Claims

1. A compound of the formula I 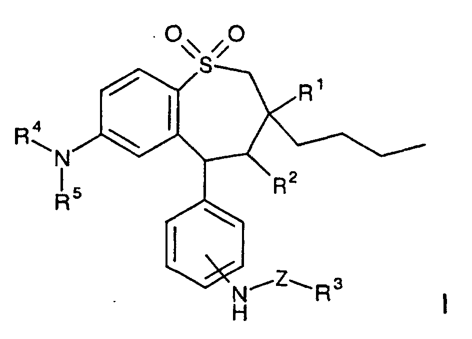 in which
R¹ is ethyl, butyl;
R² is OH;
R³ is a diamino acid radical, the diamino acid radical optionally being mono- or polysubstituted by an amino protective group;
R⁴ is methyl;
R⁵ is methyl;
Z is -(C=O)-C₀-C₄-alkyl, a covalent bond;
and its pharmaceutically tolerable salts.

2. A process for the preparation of compounds of the formula I as claimed in claim 1, which comprises reacting, according to the following equation, 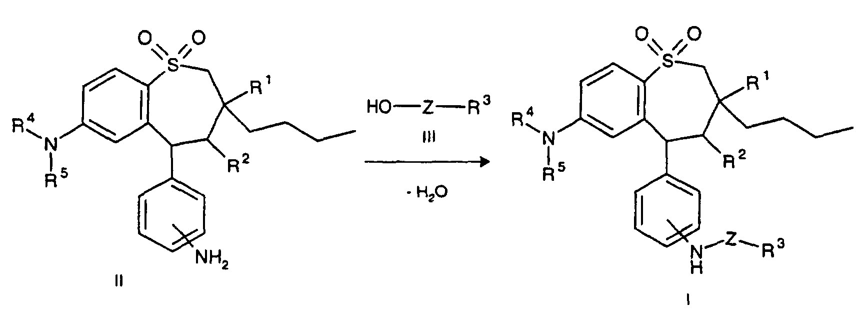 an amine of the formula II, in which R¹, R², R⁴ and R⁵ have the meanings indicated for formula I, with a compound of the formula III, in which R³ and Z have the meanings indicated for formula I, with elimination of water to give a compound of the formula I and optionally converting the compound of the formula I obtained into a physiologically tolerable salt.

3. A pharmaceutical comprising one or more of the compounds as claimed in claim 1.

4. A pharmaceutical comprising one or more of the compounds as claimed in claim 1 and one or more statins.

5. A compound as claimed in claim 1 for the production of a medicament for the treatment of lipid metabolism disorders.

6. A process for the production of a pharmaceutical comprising one or more of the compounds as claimed in claim 1, which comprises mixing the active compound with a pharmaceutically suitable excipient and bringing this mixture into a form suitable for administration.

7. The use of the compounds as claimed in claim 1 for the production of a medicament for influencing the serum cholesterol level.

8. The use of the compounds as claimed in claim 1 for the production of a medicament for the prevention of arteriosclerotic symptoms.

## Revendications

1. Composés de formule 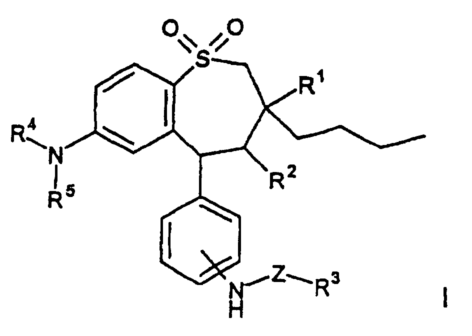 dans laquelle
R¹ représente un éthyle, butyle ;
R² représente OH ;
R³ représente un reste de diamino-acide, dans lequel le reste de diamino-acide est éventuellement substitué une ou plusieurs fois par un groupe de protection d'amino-acides ;
R⁴ représente un méthyle ;
R⁵ représente un méthyle ;
Z représente -(C=O)-alkyle en C₀-C₄, une liaison covalente ;
ainsi que leurs sels pharmaceutiquement acceptables.

2. Procédé pour la préparation de composés de formule I, selon la revendication 1, **caractérisé en ce qu'**on met à réagir selon le schéma de formules suivant 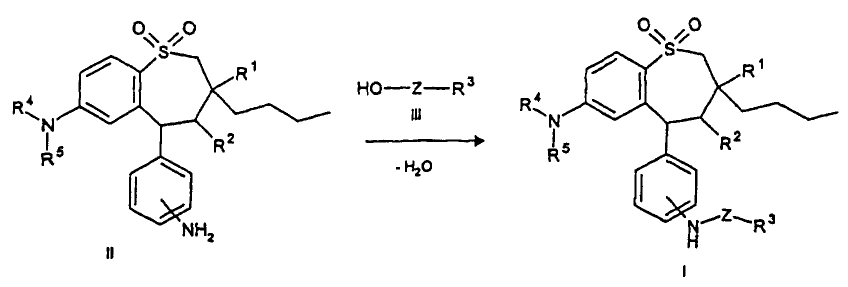 une amine de formule II, dans laquelle R¹, R², R⁴ et R⁵ ont les significations indiquées à la formule I, avec un composé de formule III, dans laquelle R³ et Z ont les significations indiquées à la formule I, par élimination d'eau pour obtenir un composé de formule I et on transforme le composé obtenu de formule I éventuellement en un sel physiologiquement acceptable.

3. Médicament contenant un ou plusieurs des composés selon la revendication 1.

4. Médicament contenant un ou plusieurs des composés selon la revendication 1 et une ou plusieurs statines.

5. Composés selon la revendication 1 pour la préparation d'un médicament pour le traitement de troubles d'échange des substances lipidiques.

6. Procédé pour la préparation d'un médicament contenant un ou plusieurs des composés selon la revendication 1, **caractérisé en ce que** la substance active est mélangée avec un véhicule pharmaceutiquement approprié et **en ce qu'**on met ce mélange sous une forme appropriée pour l'administration.

7. Utilisation des composés selon la revendication 1 pour la préparation d'un médicament pour le traitement du taux de cholestérol sérique.

8. Utilisation des composés selon la revendication 1 pour la préparation des médicaments pour la prévention des troubles artériosclérotiques.
